(19) Europäisches Patentamt
European Patent Office
Office européen des brevets

(11) **EP 2 120 975 B1**

(12) **EUROPÄISCHE PATENTSCHRIFT**

(45) Veröffentlichungstag und Bekanntmachung des Hinweises auf die Patenterteilung:
**23.09.2015 Patentblatt 2015/39**

(21) Anmeldenummer: **07857105.6**

(22) Anmeldetag: **21.12.2007**

(51) Int Cl.:
*A61K 39/02* (2006.01)      *C12N 5/078* (2010.01)
*A61K 39/00* (2006.01)      *A61K 35/14* (2015.01)

(86) Internationale Anmeldenummer:
**PCT/EP2007/011398**

(87) Internationale Veröffentlichungsnummer:
**WO 2008/077638 (03.07.2008 Gazette 2008/27)**

(54) **VOLLBLUTKULTUREN MIT STIMULIERTEN IMMUNZELLEN UND DEREN VERWENDUNG ALS HEILMITTEL**

WHOLE BLOOD CULTURES COMPRISING STIMULATED IMMUNE CELLS, AND USE THEREOF AS MEDICAMENTS

CULTURES DE SANG TOTAL À CELLULES IMMUNITAIRES STIMULÉES ET UTILISATION DE CELLES-CI COMME AGENTS THÉRAPEUTIQUES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.12.2006 DE 102006061315
25.05.2007 DE 102007024609**

(43) Veröffentlichungstag der Anmeldung:
**25.11.2009 Patentblatt 2009/48**

(73) Patentinhaber:
• **Kief, Horst**
**67069 Ludwigshafen (DE)**
• **Kief, Thorsten**
**1430 Rebecq (BE)**

(72) Erfinder: **Kief, Horst**
**67069 Ludwigshafen (DE)**

(74) Vertreter: **Wagner, Jutta et al**
**Patentanwälte**
**Zellentin & Partner**
**Rubensstrasse 30**
**67061 Ludwigshafen (DE)**

(56) Entgegenhaltungen:
**EP-A- 0 607 593      EP-A- 1 555 026**

• **COULON V ET AL: "In vitro immunization of patient T cells with autologous bone marrow antigen presenting cells pulsed with tumor lysates." INTERNATIONAL JOURNAL OF CANCER. JOURNAL INTERNATIONAL DU CANCER 1 DEC 2000, Bd. 88, Nr. 5, 1. Dezember 2000 (2000-12-01), Seiten 783-790, XP002477893 ISSN: 0020-7136**
• **WANG XU ET AL: "A comparison of folic acid and 5-methyltetrahydrofolate for prevention of DNA damage and cell death in human lymphocytes in vitro." MUTAGENESIS, Bd. 18, Nr. 1, Januar 2003 (2003-01), Seiten 81-86, XP002477894 ISSN: 0267-8357**
• **DABROSIN C ET AL: "Protection by alpha-tocopherol but not ascorbic acid from hydrogen peroxide induced cell death in normal human breast epithelial cells in culture." FREE RADICAL RESEARCH SEP 1998, Bd. 29, Nr. 3, September 1998 (1998-09), Seiten 227-234, XP009099098 ISSN: 1071-5762**

**Beschreibung**

Die vorliegende Erfindung betrifft Vollblutkulturen mit stimulierten immunkompetenten Zellen, deren Herstellung und diese enthaltende Heilmittel.

Da maligne Tumoren über aus dem Zellverband losgelöste Einzelzellen und Zellgruppen hämatogen und lymphogen metastasieren, böte eine Eigenblutkultur im Prinzip eine Ideale Möglichkeit spezifisch gegen Tumorzellen aktivierte Killerzellen zu züchten. Das selektive Abtöten von Tumorzellen in einer derartigen Kultur kann relativ einfach durch einen Sauerstoffüberschuß in der Kulturatmosphäre erfolgen. Da Tumorzellen durch die teilweise oder totale Umstellung auf Gärungsstoffwechsel relativ intolerant gegen aktive Sauerstoffstufen sind, werden sie im Gegensatz zur normalen gesunden Blutzelle früher abgetötet. Diese toten Tumorzellen oder deren Bruchstücke dienen sodann als Antigen beim spezifischen Erkennungsprozeß, der sich in einer derartigen Kultur entwickelnden Abwehrzellen. Nicht nur der Antigenerkennungs- und -verwertungsprozeß erfordert nahezu das gesamte Spektrum von Lymphozyten und Monozyten, sondern auch das Wechselspiel der Zytokine erfordert die passenden Rezeptoren eines großen Spektrums immunkompetenter Zellen, die nur eine Vollblutkultur liefern kann.

Neben dem Sensibilisierungsprozess immunkompetenter Zellen gegen Tumorantigene in einer derartigen Kultur bietet die Neuerung auch die Möglichkeit virale und bakterielle Antigene sowie Allergene, wie weiter unten beschrieben, spezifisch zu selektieren und als Immunstimulanzien einzusetzen.

Wird eine derartige Kultur mit Sauerstoffüberschuß (10000 bis 30000 Pascal) in der Kulturatmosphäre beschickt, ist jedoch auch die Lebensdauer der gesunden Blutzelle, ob rot oder weiß, zwangsläufig begrenzt. Als eine ideale Schutzmaßnahme in dieser Hinsicht hat sich erfindungsgemäß Vitamin E erwie sen. Da Alphatocopherol als rein fettlösliches Vitamin (auch als Acetat) sich jedoch in einem Kulturmedium, das einer RPM-Lösung (Standardmedium für Zellkulturen) entspricht, nicht oder nur ungenügend löst, muss es einem derartigen Kulturmedium als ein wasserlösliches Emulgat zugemischt werden. Als ideale Emulgatoren erweisen sich aufgrund ihrer physiologischen Nähe zur humanen Zellmembran Phospholipide.

Folsäure als nukleophiler Baustein kann ein wesentlicher Bestandteil eines Nährmediums sein. Im vorliegenden Falle erweist sie sich überraschender Weise jedoch als zusätzlicher Emulgator und damit als sehr wertvoller Lösungsvermittler für Vitamin E. Das gleiche gilt für niedermolekulare Heparine (Molekulargewicht 1.200 bis 12.000 Dalton), aber auch andere Proteoglykane, z.B. Pentosanpolysulfat, dem selbst gemäß hiesiger Erfahrung ein zellproliferativer Effekt zugesprochen werden muss.

Das Kulturmedium (Vol: 30 - 40 ml auf 120 ml Vollblut) besteht dabei aus der typischen mit Vitaminen angereicherten Elektrolyt- und Aminosäurelösung mit 100 mg Vitamin E Acetat und 10 % Phospholipiden, davon 90 % Phosphatidylcholin, 10 mg Folsäure und 100 mg Pentosanpolysulfat.

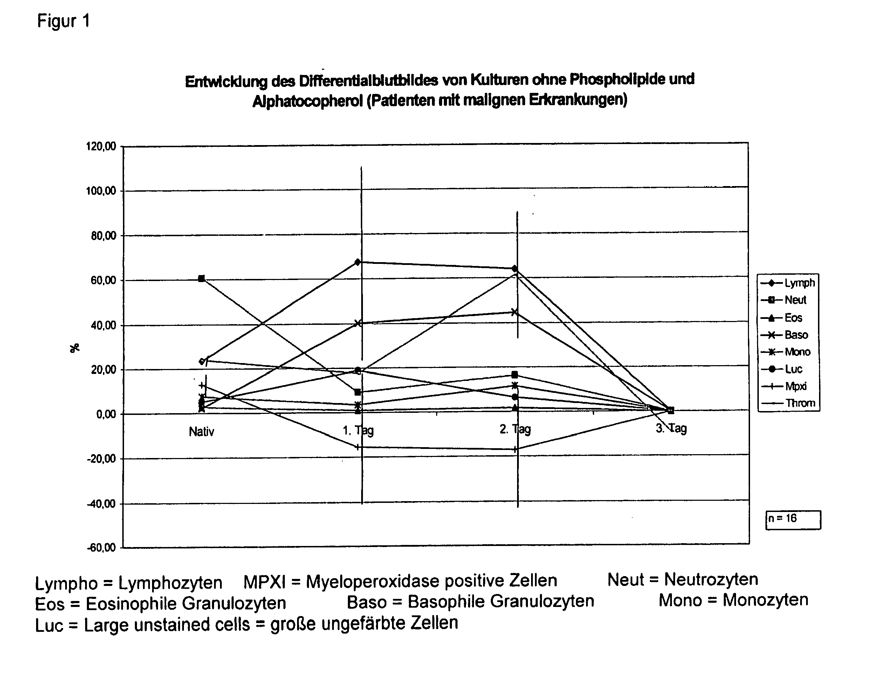

Während eine Vollblut-Kultur ohne die genannten Zusätze maximal 3 Tage überlebt (siehe Figur 1), proliferiert eine Kultur aus Vollblut mit den genannten Zusätzen über viele Tage länger mit einem durchschnittlichen Maximum von etwa 100 000 Zellen (Leukozyten) pro Mikroliter am 6. Tag.

Eine Vollblutkultur einschließlich der Erythrozyten ist aufgrund der geringen Resistenz der Erythrozytenmembran gegenüber aktivierten Sauerstoffstufen nicht überlebensfähig. Die beschriebene Neuerung hat dagegen nicht nur den Vorteil der längeren Kultivierung und damit höheren Ausbeute, sondern auch die Möglichkeit der Reaktion auf erythrozytenständige Antigene sowie der vereinfachten Selektion von Leukozytensubpopulationen wie weiter unten beschrieben.

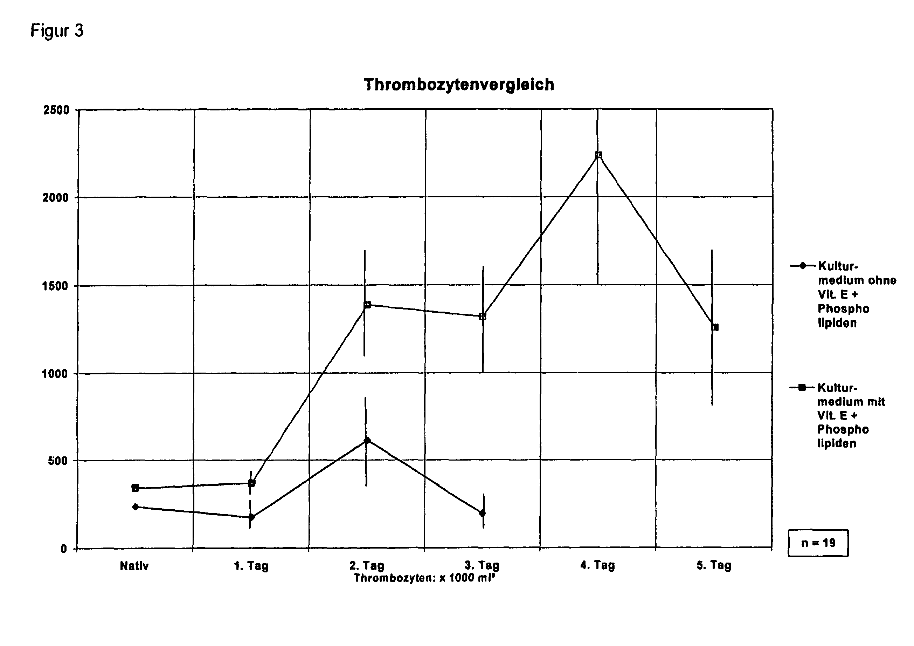

Die Thrombozyten als besonders sensibles Substrat für proliferationsstimulierende Maßnahmen steigen von etwa 600 000 am 2. Kulturtag in einem Kulturmedium ohne die genannten Zusätze auf annähernd 2,3 Mio., am 4. Kulturtag in einem Kulturmedium mit den genannten Zusätzen (siehe Figur 3).

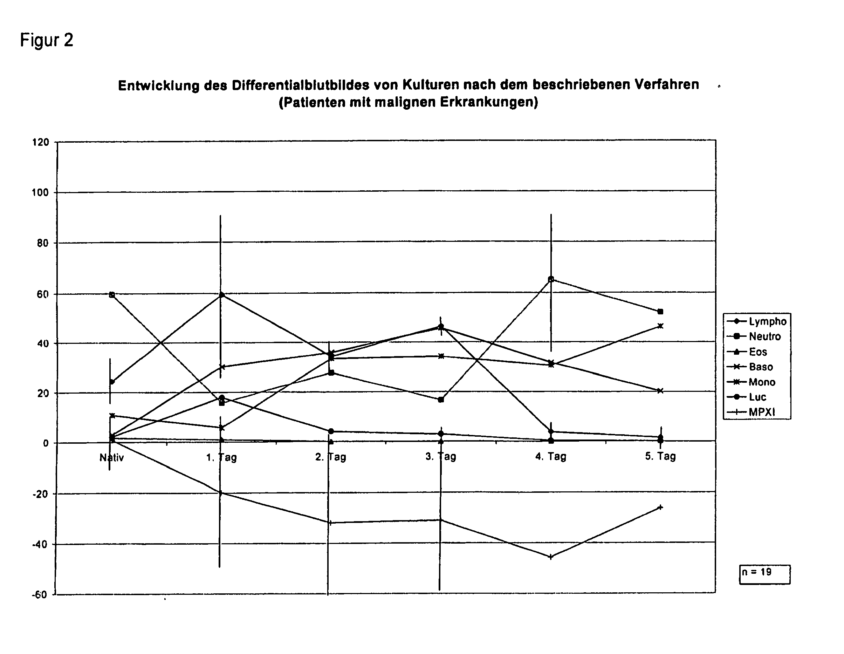

Als völlig überraschend hat sich dabei herausgestellt, dass nicht nur die Lebensdauer einer derartigen Kultur und damit die Zellausbeute wesentlich erhöht werden kann, sondern auch ein ungewöhnliches Spektrum von myeloischen Abwehrzellen mit einem außerordentlich hohen Anteil an Makrophagen entsteht ohne vorherige Selektion etwa durch Zonenzentrifugation. Wie aus der obigen Statistik und Graphik ersichtlich, entstehen am 3. bis 5. Tag einer derartigen Kultur etwa 30 bis 50 % Monozyten und etwa 35 bis 45 % Basophile Zellen (siehe Figur 2). Alle Zellzahlen wurden durchflusszytometrisch mit dem Gerät Advia 120 der Firma Bayer Technics ermittelt.

Wie aus dem Verlauf der Entwicklung des Differentialblutbildes unter den beschriebenen Kultivierungsbedingungen ersichtlich, steigt die Proliferation der myeloischen Reihe am 4. und 5. Tag bei gleichzeitigem Absinken der Lymphozyten stark an. Wird zu diesem Zeitpunkt geboostert mit weiterer Zugabe eines Vitamin E - Proteoglykan-Gemischs, dann steigt die Anzahl der Leukozyten unter besonderer Betonung der Monozyten und Basophilen auf eine Höhe an, die die bisherige Erfahrungen in der Hämatologie übertreffen. Es wird dann eine Zelldichte bis zu mehreren hunderttausend Leukozyten pro Kubikmillimeter erzielt.

Basophile Leukozyten sind als sehr gut ausgestattete Effektorzellen gewissermaßen das Endprodukt der zellulären myeloischen Abwehr. Auch der sehr hohe Anteil an Thrombozyten, die unter anderem in einer derartigen Kultur auch als Riesenthrombozyten auftreten, spricht für einen potenzierten Antigen-Antikörper-Erkennungsprozeß und auch eine erhöhte Makrophagentätigkeit, die diesen Zellen unter anderem ebenfalls zuerkannt wird (siehe Figur 3). Um diesen Prozess zu forcieren, genügen Phospholipide aus der Sojabohne als Gemischanteil (siehe Zeile 40-44). Dies ist einerseits überraschend, da Sojaphospholipide in ihrer mengenmäßigen Verteilung nur bedingt etwa der strukturellen Verteilung der Phospholipide der humanen Zellen oder den Phospholipiden bspw. von Bakterienwänden, insbesondere von gramnegativen Bakterien entsprechen, denen ein eindeutiger Antigencharakter mit Induktion bspw. von Tumornekrosefaktor zuerkannt wird. Sojaphospholipide haben darüber hinaus den Vorteil, dass sie bei parenteraler Gabe praktisch untoxisch sind, daher sogar grammweise intravenös verabreicht werden können, was bspw. auf MPL, (Monophosphoryl-Lipid A, Derivat des Lipopolysaccharids einer Salmonellenart) das als Immunadjuvans bei der Hyposensibilisierung eingesetzt wird, nicht zutrifft. Es kann daher nur in Dosen bis zu 50 mg verabreicht werden.

Beim Vergleich einer Standardkultur mit einer Vollblutkultur mit Phospholipidstimulation stellt man fest, dass die Lymphozyten zugunsten der myeloischen Reihe absinken, die basophilen Granulozyten prozentual um das doppelte höher liegen, die Monozyten gar um das 4- bis 5-fache ansteigen und die Thrombozyten vergleichsweise einen extremen Anstieg zu verzeichnen haben. (siehe Figur 2 und 3)

Werden Makrophagen aktiviert, entwickeln sie Agglutinine und haften damit an der Glaswand des Reaktionsgefäßes. Üblicherweise werden aktivierte Makrophagen durch Trypsinlösungen von der Glaswand abgelöst. Neuerungsgemäß erfolgt dies besser mit Haeslösung (Hydroxyethylstärke), da dieser Prozess wesentlich schonender abläuft und zu einer höheren Überlebensrate der Zellen führt.

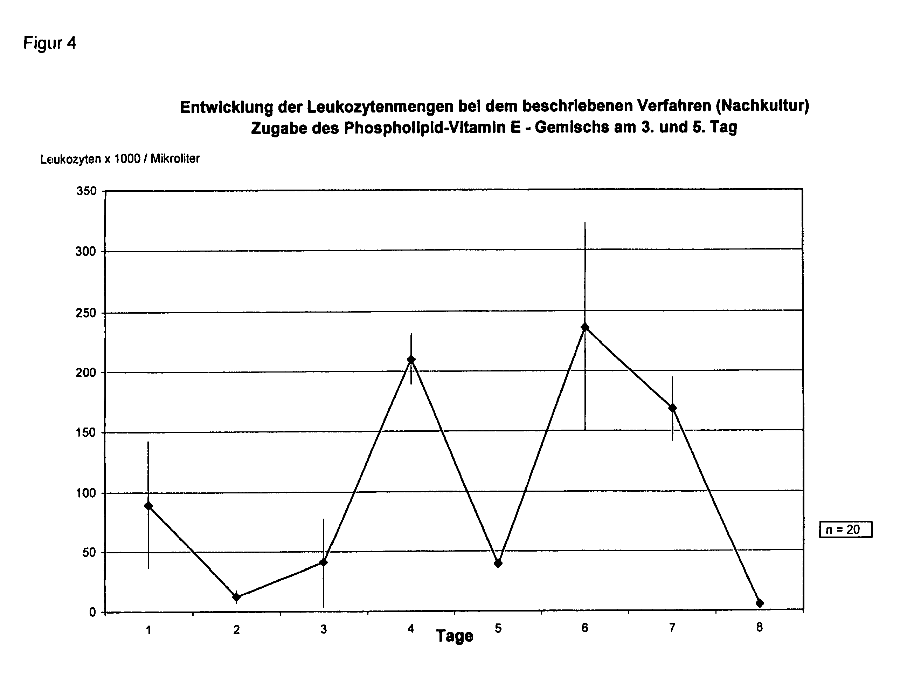

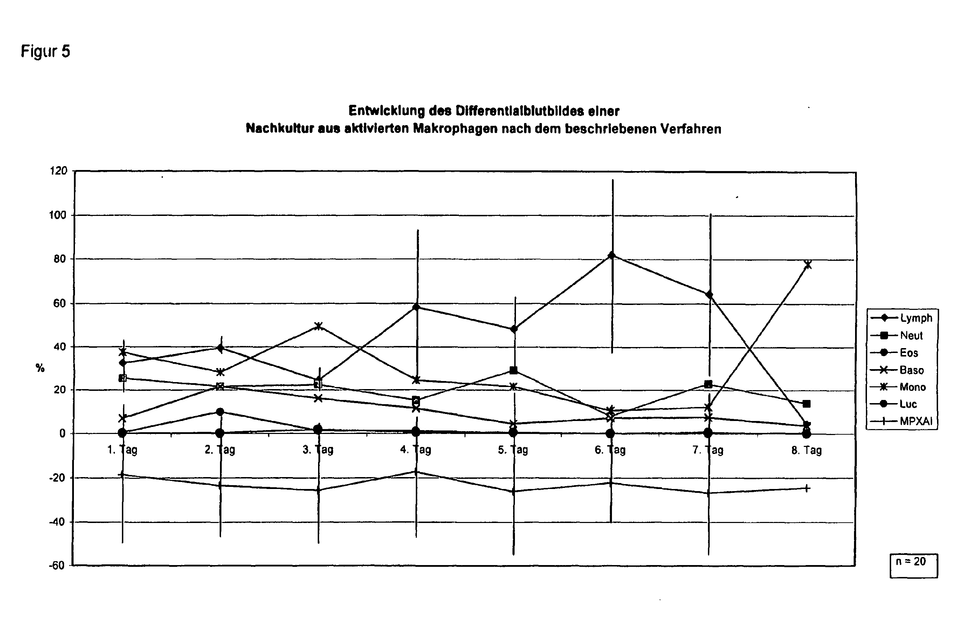

Wird eine derartige Zweitkultur nach dem beschriebenen Kulturverfahren stimuliert, entstehen durchschnittlich 250 000 Leukozyten pro Mikroliter (Figur 4), bei einem außerordentlich hohen Anteil von Monozyten am 8. Tag (Figur 5).

Das Kulturmedium ist in der Basis aus den Bestandteilen zusammengesetzt, die eingangs in den Absätzen [0004] bis [0006] beschrieben wurden. Als Serumzusatz dient das Spenderplasma, das mit Ozon von einer Konzentration von 50 - 70 $\mu$g bei doppeltem bis vierfachem Volumenanteil (Plasma zu $O_2$-$O_3$-Gemisch wie [1:2 (4)] behandelt wurde. Der Vorteil eines solchermaßen aufbereiteten Kulturmediums liegt in der Verträglichkeit des Mediums zu den autologen Spenderzellen, der Funktion des ozonisierten Plasmas als $O_2$-Donator (bis über $5,6 \times 10^5$ bar!) und der Freiheit von autogenen Keimen durch die Ozonisierung. Darüber hinaus ist es der ideale Ersatz für bovines Kulturserum, das überlicherweise bei Zellkulturen verwendet wird, da es garantiert prionenfrei ist.

Die besonders hohe Ausbeute an Leukozyten am 4. und 6. Tag ist bei einer derartigen Kultur bedingt durch die Zugabe der beschriebenen Proliferationsstimulanzien am 3. und 5. Tag (Figur 4).

Aus den Monozyten einer derartigen Zweitkultur lassen sich auf bekannte Art und Weise nach dem Stand der Technik dendritische Zellen gewinnen, die sich weltweit in Pilotstudien als relativ wirksam gegen maligne Erkrankungen erwiesen haben.

Wird der beschriebene Vorgang des Starts einer neuen Kultur über aktivierte Makrophagen, die an der Wand des Reaktionsgefäßes haften, erneut vorgenommen, lässt sich der Anteil der Monozyten noch weiter steigern. Der Vorteil des Verfahrens liegt nicht nur darin, aus dieser Zellpopulation monozytenabgeleitete dendritische Zellen zu gewinnen, sondern auch gleichzeitig die Partner der Antigenerkennung und Verwertung, die Lymphozyten, mit anzubieten (siehe Figur 5).

Die Kontrolle der Lymphozytensubpopulationen ergab dabei einen T-Zell-Gehalt bis zu 30 000/mm$^3$, woraus sich wiederum auf einen gewissen "Antigenboostereffekt" des Phospholipid-Vitamin E- Proteoglykangemischs schließen lässt.

Ein solches Präparat, aus einer Eigenblutkultur gewonnen, kann enteral und parenteral aufgrund seiner autologen Herkunft praktisch ohne Nebenwirkungen verabreicht werden. Trotz der qualitativen und quantitativen Änderungen der Eigenblutbestandteile kann ein derartiges Präparat intravenös bis zu mehreren Millilitern bei entsprechender Verdünnung verabreicht werden.

Ein Heilmittel gemäß der beschriebenen Neuerung kann aufgrund seiner Steuerungsimpulse auf das Knochenmark außerdem mit guter Aussicht auf Erfolg bei Leukopenie, Thrombopenie und zellulären Immundefekten eingesetzt werden, insbesondere bei jenen, die eine erhöhte Makrophagenaktivität erfordern.

Der außerordentliche Reichtum an Monozyten und deren besonderen Abkömmlingen, den dendritischen Zellen, bei derartigen Kulturen stellt eine ideale Voraussetzung für die Präsentation spezifischer Antigene dar. Beschickt man daher eine solche mit Monozyten angereicherte Eigenblutkultur mit Antigenen, bspw. attenuierten Viren, Polysacchariden oder (Ultra-) Zentrifugaten aus Bakterien oder Pilzen oder Zellfraktionen aus malignen Zellen autologer oder homologer Herkunft, ist eine enorme Steigerung der körpereigenen spezifischen Abwehr je nach dem gewählten Antigen zwangsläufig. Zweckmäßigerweise können derartige Antigene gewonnen werden aus Bakterienkulturen, die aus Eigenblut, Urin oder Stuhl stammen. Derartige Keime können nach Lyophilisation, Prüfung auf Vermehrungsfähigkeit und Sterilität einer Eigenblutkultur als Stimulans zugegeben werden, so dass nach Abschluss der Kultivierung ein Blutprodukt als Impfstoff zur Verfügung steht, das bspw. bei therapieresistenten Infekten mit sehr guten Erfolgsaussichten eingesetzt werden kann. Insbesondere Autoimmunprozesse, die durch sogenannte Fokalinfekte hervorgerufen wurden, sprechen auf derartige Präparate sehr gut an.

Werden die beschriebenen Antigene allein oder in Kombination unter Beimengung des Phospholipid/Vitamin E Gemischs der Kultur zugegeben, tritt ein Potenzierungseffekt auf die Zellproliferation ein, der dann in extremen Zellzahlen bis zu 900 000 Leukozyten / Mikroliter gipfelt.

Gibt man zu einer derartigen Vollblut-Kultur einen halben Milliliter eines standardisierten Lysates von Diplococcus pneumoniae, Haemophilus influenzae, Streptococcus pyogenes und faecalis, Staphylococcus aureus, Klebsiella pneumoniae, Neisseria catarrhalis, flava und perflava, Gaffkya tetragena und Moraxella, entsprechend einer Gesamtmasse von 7,5 Milliarden Keimen, mit einem Endotoxingehalt von über 10 000 Einheiten (gemessen im Limulustest), lassen sich nach Kulturabschluß deren Endotoxine nicht mehr nachweisen, ein ebenso überraschender wie deutlicher Hinweis auf die ungewöhnlich gesteigerte Makrophagentätigkeit, die sich mit einer derartigen Vollblut-Kultur erzielen lässt.

Eine weitere individualspezifische Immunreaktion ist zu erzielen durch Beschicken dieser Eigenblutkulturen mit Allergenen, wie sie in klinischen Tests wie z.B. Rast-Test oder einschlägigen Labortests ermittelt werden können. Üblicherweise werden derartige Allergengemische in Form der so genannten Hyposensibilisierung zur Entwicklung einer Toleranz im menschlichen Organismus eingesetzt. Diese Therapie, deren Wirksamkeit in zahlreichen kontrollierten Studien belegt ist, hat jedoch zwei entscheidende Nachteile. Erstens: Sie muss über Jahre durchgeführt werden und zweitens beinhaltet sie durch den unmittelbaren Kontakt des Organismus mit dem Allergen die prinzipielle Gefahr des Schocks mit manchmal prekärem Ausgang.

Eine Eigenblutkultur der beschriebenen Art ist aufgrund ihres Reichtums immunkompetenter Zellen geradezu prädestiniert zur Produktion von spezifischen Immunglobulinen G, die gemäß unserem derzeitigen Wissensstand verantwortlich zeichnen für die Entwicklung einer Toleranz. Ein derartiges Präparat kann daher mit guter Aussicht auf Erfolg ohne die beschriebenen Nachteile der Hyposensibilisierung eingesetzt werden.

Der therapeutische Einsatz bakterieller Antigene z. B. in Form abgetöteter Bakterien gegen maligne Erkrankungen ist seit Beginn des vorigen Jahrhunderts bekannt. Auch kennt man den Einsatz von BCG Stämmen bei verschiedenen Formen maligner Erkrankungen, unter anderem auch in Zuchten dendritischer Zellen.

In zahlreichen Pilotstudien wird über ein unterschiedliches Ansprechen von Präparaten derartiger Herkunft berichtet. Die unmittelbare Anwendung von Bakteriensuspensionen am Patienten ist verständlicherweise nicht ungefährlich und per se mit erheblichen Nebenwirkungen behaftet.

Auch die Anwendung von Eigenblutkülturen, die mit lebenden Bakterien konfrontiert werden, ist mit nicht unerheblichen Risiken behaftet. Verwendet man dagegen exakt definierte Bestandteile aus Viren, wie sie bspw. bei Impfstoffen Verwendung finden (Agglutinine und Muraminidase) oder Lyophilisate und Ultrazentrifugate aus Bakterien oder Pilzen z.B. Kernbestandteile oder Ribosomen, bestehen diese Risiken nicht. Derartige Präparate werden auch als Fertigarzneimittel auf dem Arzneimittelmarkt angeboten.

Während man aufgrund derzeit gängiger Testverfahren bei Allergien ein einigermaßen sicheres Antigenmuster zur Hyposensibilisierung zur Verfügung hat, ist dies zur Stimulation derartiger Kulturen bei bakteriellen oder viralen Erregern bislang entweder nicht möglich oder nur mit einem sehr hohen Laboraufwand, bspw. mittels serologischer Proben zu realisieren.

Das beschriebene Verfahren bietet dagegen eine relativ einfache Möglichkeit, die Sensibilität auf Antigene zu testen. Durch den eingangs erwähnten Sauerstoffüberschuss, insbesondere in Form aktivierter Sauerstoffstufen, werden die weißen Blutkörperchen gegenüber Antigenen erhöht empfindlich und ändern daher ihre Größe, ihre Zahl als auch die Menge an anfärbbarem Biomaterial im Zellinneren. Diese Parameter lassen sich einfach in modernen Automaten (so genannte Zellcounter) zur Zelldifferenzierung des weißen Blutbildes in den einzelnen Meßkanälen überprüfen. Wird daher ein derartiger Kulturansatz mit dem jeweiligen Antigen konfrontiert, dann variiert die Beladung der Neutrophilen mit anffärbbarem Biomaterial (Neut X) ebenso die zelluläre Dichte der Neutrophilen (Neut Y) und das Volumen der "geschrumpften" Leukozyten (IMI DC) (siehe Betriebsanleitung für die Zellcounter der Firmen Sysmex oder Beyer Technics).

Die Monozyten als antigenerkennende Zellen nehmen in der Regel ab, können jedoch in besonderen Fällen und bei entsprechender Kulturdauer auch in ihrer Anzahl zunehmen. Die Thrombozyten als besonders sensibles Substrat reagieren auf die Toxizität des zugesetzten Antigens mit einer zahlenmäßigen Verringerung, während das Volumen der Erythrozyten entsprechend dem Toxizitätsgrad des Antigens zunimmt.

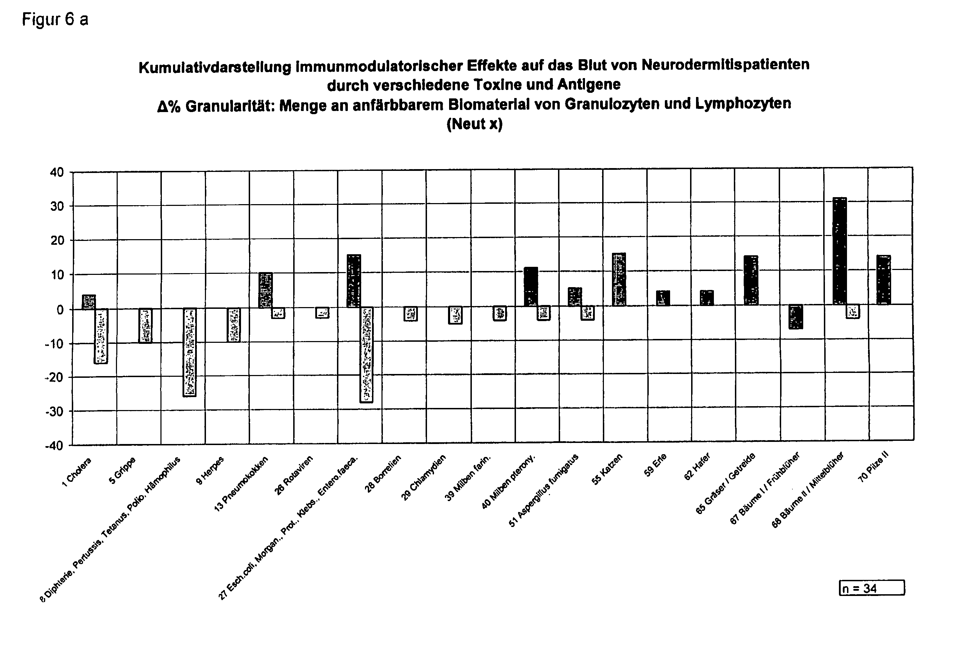

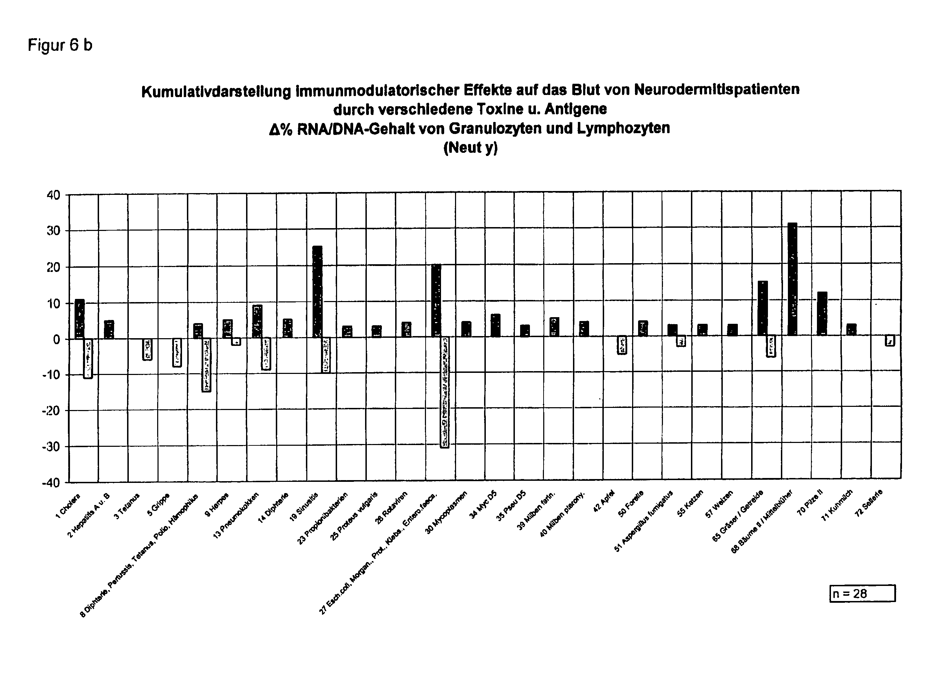

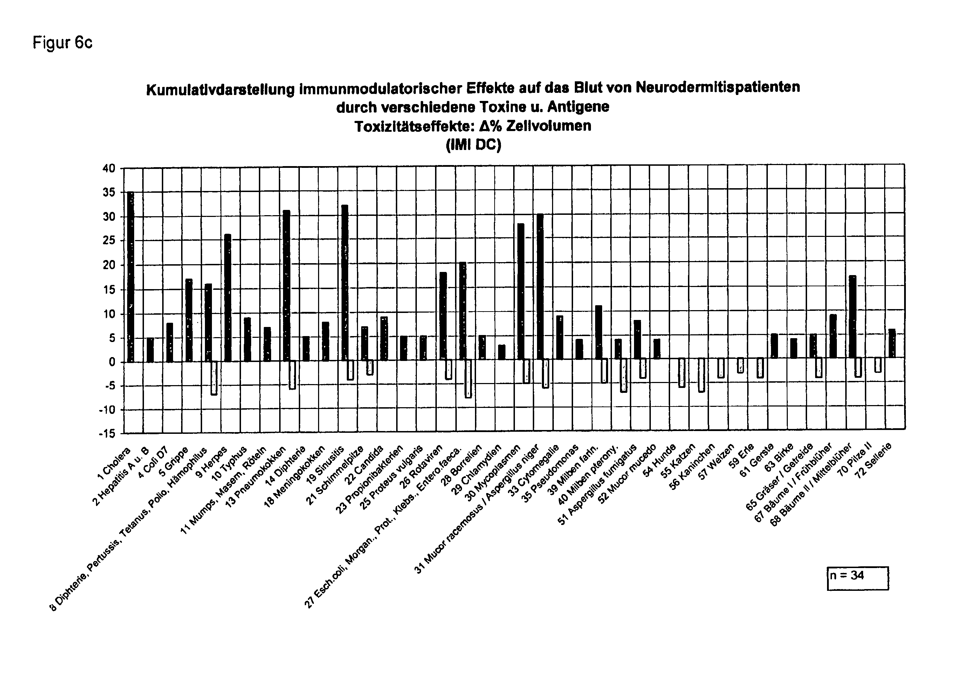

Man geht dabei so vor, dass man eine Reihe von Kulturansätzen der beschriebenen Art, Gesamtmenge an Heparin-Vollblut (500µl), oder als Citratblut mit einem $O_2$-$O_3$-Gemisch von 5 - 15 µg $O_3$ Konzentration (10ml Gas auf 20ml Blut) behandelt wurde, mit 50µl Antigenlösung, deren Konzentration empirisch ermittelt wurde, versetzt und 6 bis 12 Stunden im Inkubator oder bei Zimmertemperatur inkubiert. Danach werden diese Minikulturen über einen modernen Zellcounterautomaten differenziert. Es werden die Differenzen ermittelt zwischen der unbehandelten Kultur und der mit Antigenen beschickten Kultur und in einem graphischen Profil abgetragen (siehe Figur 6a: Neut X, Figur 6b: Neut Y und Figur 6c: IMI DC). In Figur 6c werden die antigenbedingten Schrumpfungs- und Blähungsprozesse durch den Kanal IMI DC des Zellcounters erfasst.

Aus diesen Basiswerten wird "Sensibilitätsfaktor 1" errechnet nach folgender Formel:

$$SF1 = \frac{\Delta \text{ Neut X\% x } \Delta \text{ Neut Y\% x } \Delta \text{ IMI DC\%}}{\Delta \text{ Mono\%}}$$

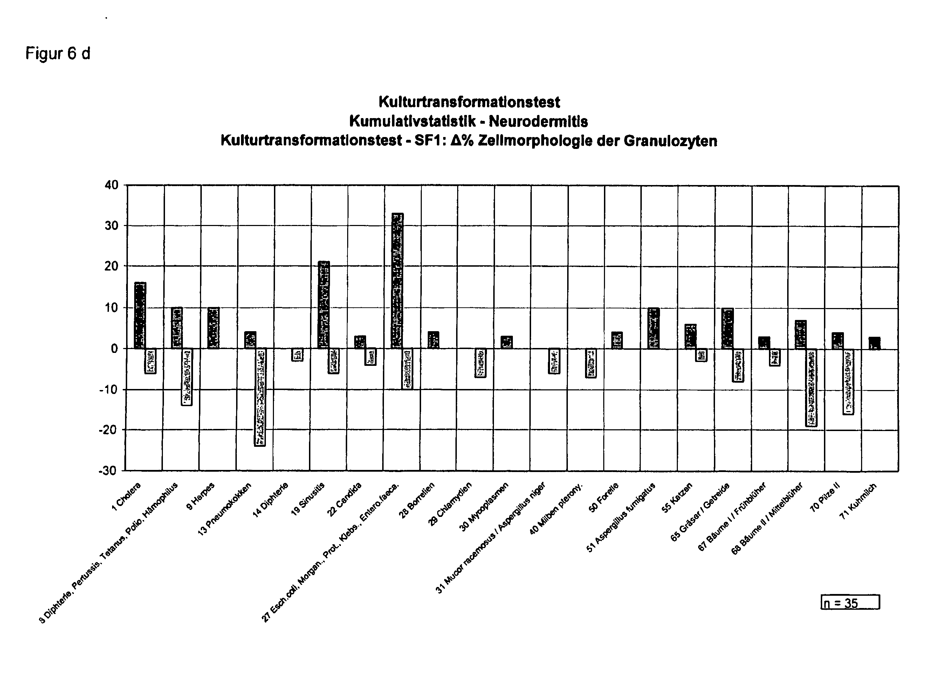

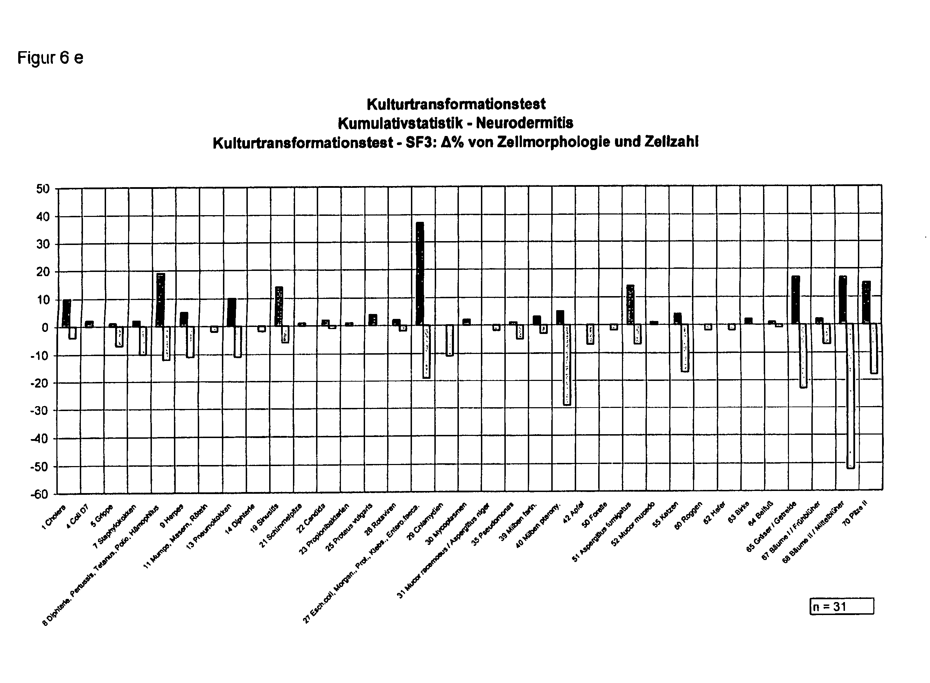

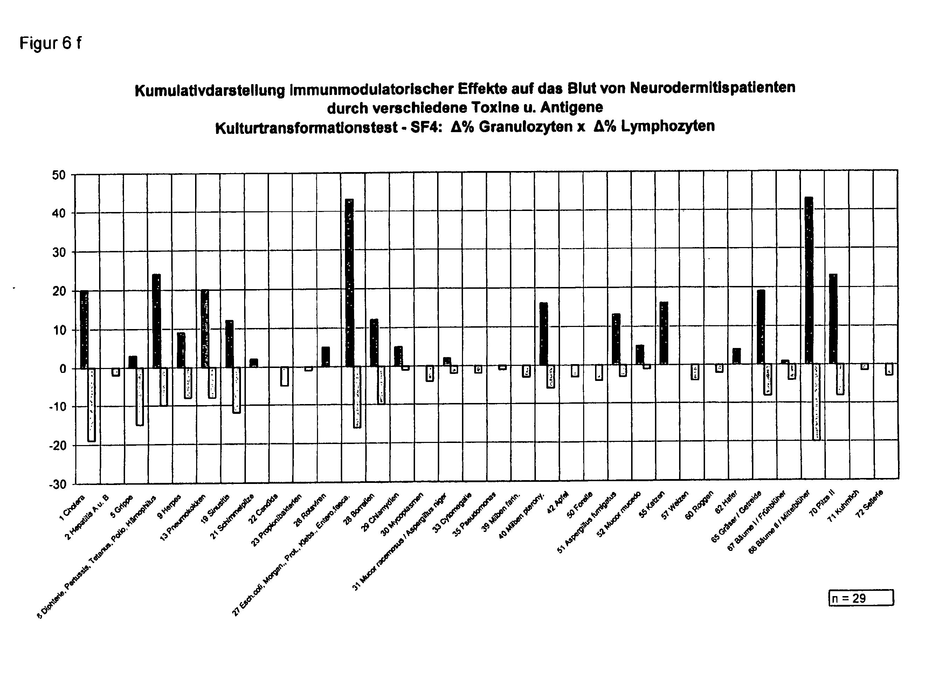

(siehe Figur 6 d), wobei "Mono" Monozyten in absoluter Zählweise bedeutet:

$$\Delta \text{ Mono\%} = \frac{(\text{n Mono blank} - \text{n Mono kontaminiert})}{\text{n Mono blank}}$$

und "Neutr" neutrophile Granulozyten; "Lympho" Lymphozyten; Neut X, Neut Y und IMI DC die Zahlen darstellen, die über die jeweiligen Meßkanäle des Countautomaten ermittelt werden. Die so ermittelten Sensibilitätsfaktoren werden in einem grafischen Profil abgetragen, aus dem sich hernach leicht eine Auswahl spezifischer Antigene aufgrund der Ausmaße der Ausschläge zur Stimulation ermitteln lässt (siehe Figuren 6a bis 6e), die die ermittelten Ergebnisse statistisch erfassen.

Bekanntermaßen besteht eine unterschiedliche Affinität von Antigenen zu spezifischen Zellen. Dies berücksichtigen die weiteren Sensibilitätsfaktoren 3 und 4 (siehe Figuren 6e und 6f), wobei Sensibilitätsfaktor 3 errechnet wird nach der Formel

$$SF3 = \frac{\Delta \text{ Neut X\% x } \Delta \text{ Neut Y\% x } \Delta \text{ IMI DC\%}}{\Delta \text{ n Neutr\% x } \Delta \text{ n Monozyten\% x } \Delta \text{ n PLT\% x } \Delta \text{ n Lympho\%}}$$

In dieser Formel stellen "n PLT", "n Neutr.", "n Lymph." und "n Mono" Thrombozyten, Neutrophile, Granulozyten, Lymphozyten und Monozyten in absoluter Zahlweise dar.

Sensibilitätsfaktor 4 wird berechnet nach der Formel:

$$SF4 = \frac{(\Delta \text{ Neut X\% + } \Delta \text{ Neut Y\% + } \Delta \text{ IMI DC\%})}{(\Delta \text{ n PLT\% + } \Delta \text{ n Neutr\% + } \Delta \text{ n Lympho\%})} \text{ x } \Delta \text{ n Mono\%}$$

Sensibilitätsfaktor 4 repräsentiert mit den Monozyten im Zähler vorwiegend die Antigenerkennung der zellulären Immunität, während SF3 das umfassendste Bild über den Einfluss der Antigene auf die Leukozyten (+ Thrombozyten) vermittelt.

Die Optimierung der Aussagekraft des Testes durch die Gewichtung einzelner Gruppen immunkompetenter Zellen lässt sich außer auf mathematischem Weg auch auf einfache Weise durch Separation einzelner Zellgruppen erzielen.

Dies wäre einerseits durch Separation in einer Zentrifuge möglich, andererseits auf relativ simple Art durch Lagerung. Wird eine derartige Testkultur wenige Tage bei Kühlschranktemperatur oder in entsprechend kürzerer Frist bei Raumtemperatur gelagert, entstehen - messbar durch den Zellcounter - freie Kernbestandteile im Kulturgut, die der Zellcounter als Granulozyten oder deren Bestandteile ausweist.

In der Regel hat dieser Prozess bei Raumtemperatur bereits nach 6 bis 48 Stunden eine ausreichende Intensität erreicht, dass in diesem Testverfahren vorwiegend Lymphozyten und Monozyten als Reaktionspartner für die Toxine auftreten.

Die Kontrolle des kontaminierten Testgutes maximal 6 Stunden nach Beimpfung ergibt also eine verwertbare Aussage unter Hinzuziehung der Granulozyten und nach zwei Tagen Lagerung eine klar differenzierte Aussage unter Betonung von Lymphozyten und Monozyten.

Die Ergebnisse dieses Testverfahrens nach mehrtägiger Lagerung bei Kühlschranktemperatur (2°C - 8°C) lassen sich folgerichtig umsetzen in der Kultivierung einer autologen Blutkultur, d.h. eine 2 - 6 tägige Kühllagerung der Blutkonserve vor Kultivierung führt zwangsläufig zu einem Überwiegen von Lymphozyten und Monozyten im Endprodukt und damit zu einer Selektion und Optimierung des therapeutischen Spektrums.

Man erkennt in Figuren 6a bis 6f, dass die Ergebnisse bei dem Patientengut Neurodermitis sich weitgehend mit den bislang bekannten klinischen Erfahrungen decken.

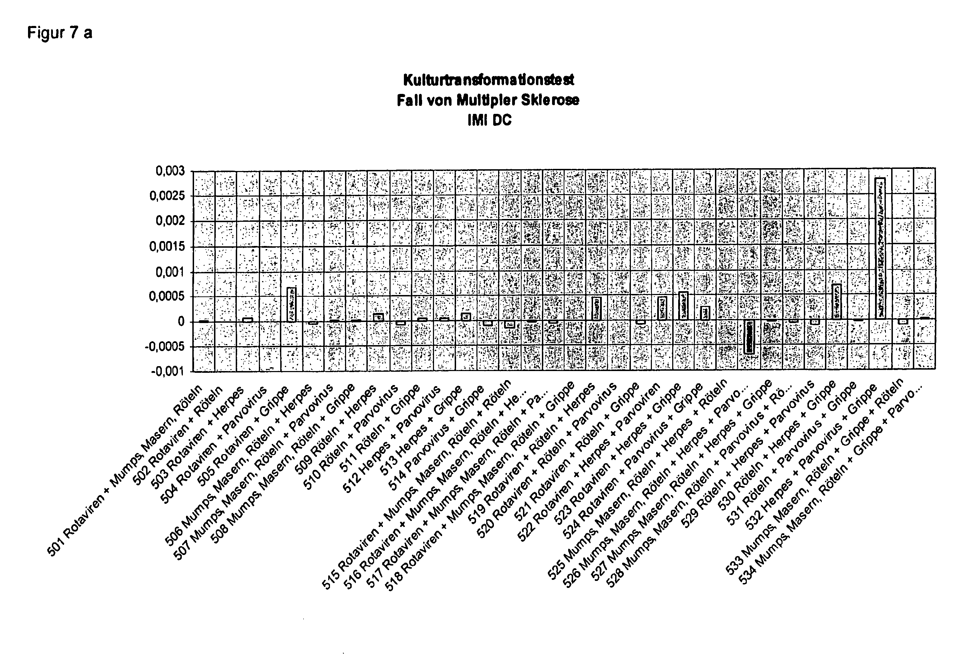

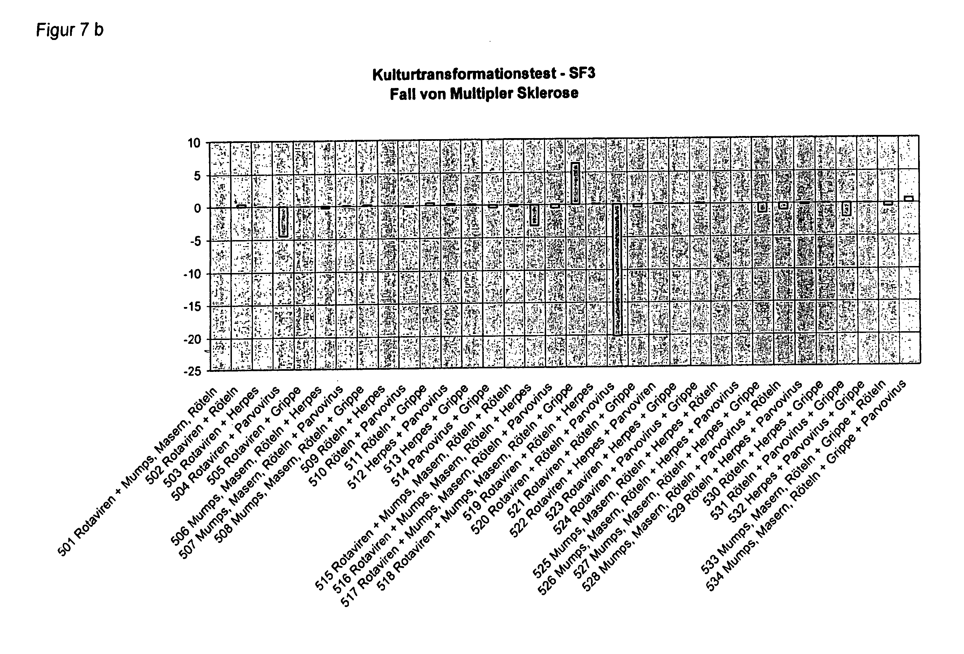

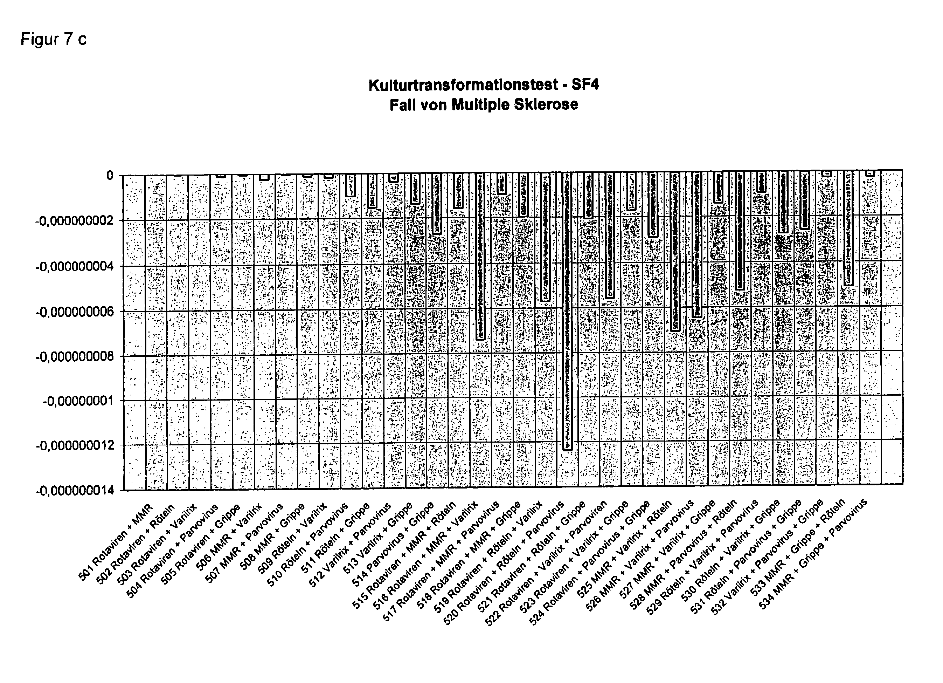

Eine Auswahl von Stimulationsfaktoren für das erfindungsgemäße Verfahren und die Beschickung von Eigenblutkulturen mit derartig ausgewählten Antigenen hat sich nicht nur diagnostisch bewährt, sondern auch klinisch zu ungewöhnlichen Erfolgen geführt. Bei der Kontrolle derartig stimulierter Kulturen traten zwei Phänomene zu Tage, die bislang unbekannt waren: Insbesondere im Bereich der Z-Achse (Toxizitätseffekte) wurde bei bestimmten Indikationen durch Kombination verschiedener Antigene ein ungewöhnlich starker toxischer Effekt erzielt, d. h. die toxische Wirkung ausgewählter Erreger zeigte in Kombination einen potenzierenden toxischen Effekt auf die immunkompetente Zelle [siehe Figur 7a, 7b und 7c: Wirkung der Kombination von Herpesviren, Rötelviren (Ziffer 509, siehe Figur 7c), dagegen bei einer Kombination von Herpesviren, Röteln und Parvoviren (Ziffer 529, siehe Figur 7b) eine Aufhebung der tox. Wirkung der Parvoviren (Konkurrenz um die Besetzung der selben Rezeptoren)].

Es gibt demnach Kombinationen von Erregern, die einen subtraktiven Effekt auf die toxische oder auch nur immunmodulatorische Wirkung durch ein Virus haben. Der Test versetzt den Fachmann demnach in die Lage, durch Auswahl spezifischer Erregerkombinationen entweder ein potenzierenden Effekt auf das Immunsystem auszuüben oder aber die Schadwirkung eines Virus durch Kombination mit einem zweiten oder weiteren zu reduzieren oder gar aufzuheben (z. B. durch Besetzung der Rezeptoren).

Die toxische Wirkung einzelner Antigene oder ausgewählter Antigenkombinationen kann dabei soweit gehen, dass eine derartige Kultur innerhalb weniger Stunden zerstört werden kann. Ein Prozess der selbst eine diagnostische Verwertbarkeit mit hoher Sicherheit zulässt, in dem bspw. das Zerstörungspotential des jeweiligen Antigens pro Zeiteinheit gemessen wird.

Wie die weiteren Figuren zeigen, lassen sich je nach Zugabe von Stimulationsmaterial in Abhängigkeit von der Kultivierungszeit unterschiedlich zusammengesetzte Zellsuspensionen gewinnen.

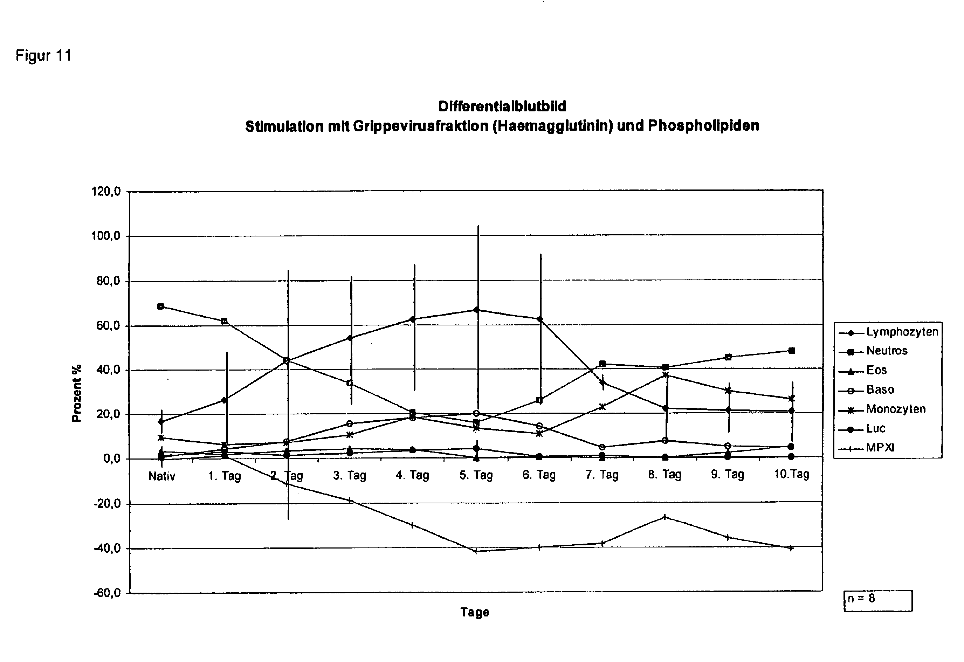

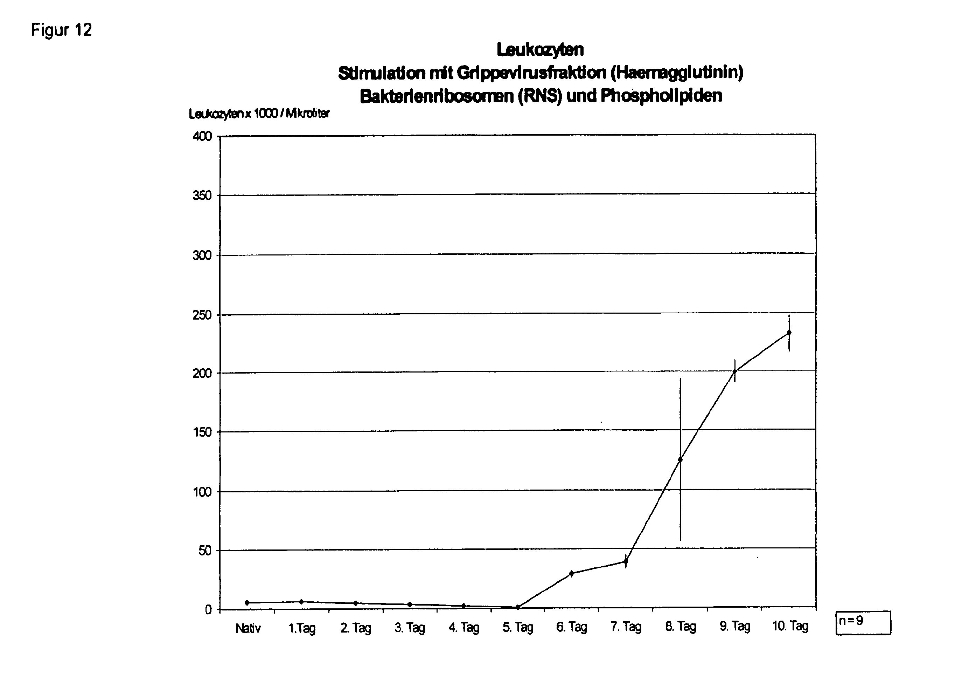

Die Nachkultur einer nach dem erfindungsgemäßen Verfahren gemäß Figur 11 und Figur 12 stimulierten Eigenblutkultur zeigt einen außerordentlichen Reichtum an Lymphozyten und bietet sich damit an zum Einsatz bei einer spezifischen Antikörpermangel-Symptomatik.

Wie aus den Verläufen der verschiedenen Subpopulationen der myeloischen Reihen ersichtlich ist, erzielen Leukozyten, Lymphozyten und andere Zellreihen je nach Art der Antigenauswahl und deren Kombination zu unterschiedlichen Zeitpunkten ihre Maximalwerte, woraus sich wiederum ergibt, die beschriebenen Stimulationen zu einer optimierten Vorlaufszeit - je nach klinischer Indikation - durchzuführen.

Wird aufgrund des Kulturtransformationstestes eine optimierte Antigenkombination z. B. bei malignen Erkrankungen gefunden, wird nicht selten ein Anstieg von eosinophilen Granulozyten in der Kultur von 40 % - 90% gefunden, was in aller Regel ein Signum für eine erfolgreiche, praktisch nebenwirkungsfreie Behandlung von Krebserkrankungen darstellt.

Der Einsatz monozytenangereicherter, mit Bakterien- oder Virusfraktionen stimulierter Kulturen bei malignen Erkrankungen führt bei vielen so behandelten Patienten zu einem sehr raschen Absinken der Tumormarker innerhalb weniger Tage, z.B. bei Pankreas-Ca, Ovarial-Ca, Colon-Ca und Prostata-Ca.

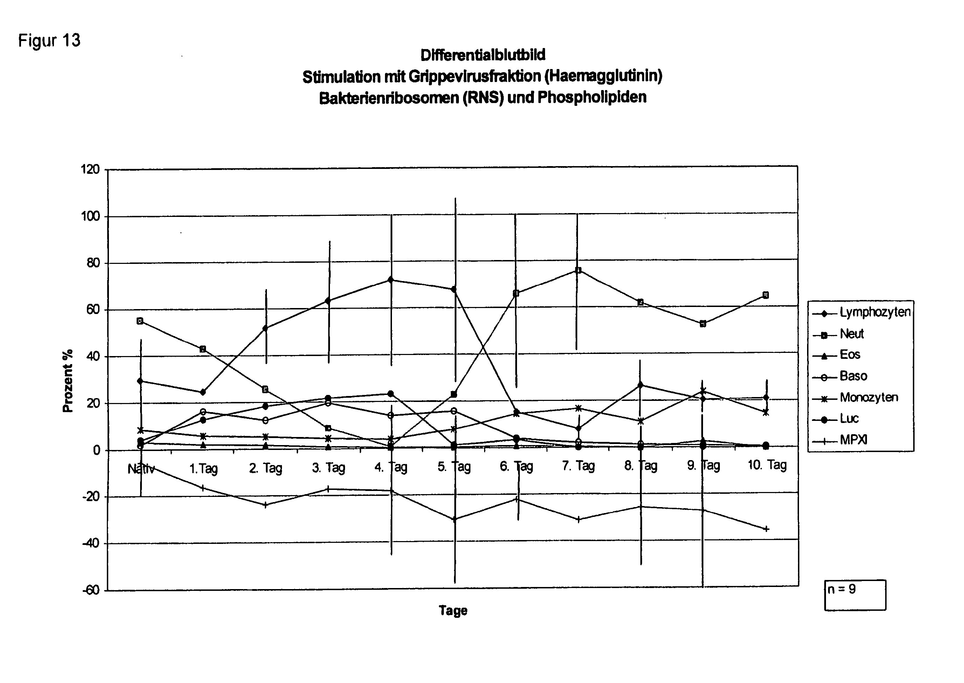

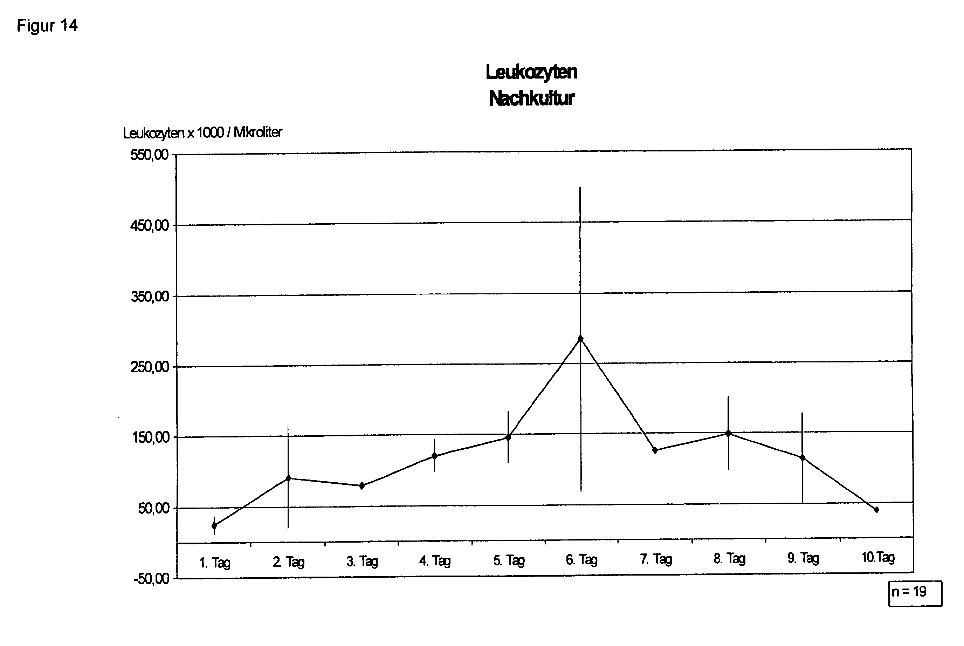

Wie die Figuren 13 und 14 zeigen und wie dies aus den Ergebnissen des Kulturtransformationstests (7b und 7c) hervorgeht, bewirkt die Zugabe von Bakterien- oder Virenbestandteilen einen vorübergehenden Abfall der Gesamtleukozytenzahl sowie einzelner Zelllinien der myeloischen Reihe.

Wird daher eine derartige Kultur bspw. mit einem plurivalenten Impfstoff kontaminiert, dann kann die Leukozytenzahl bis auf wenige hundert Zellen pro Mikroliter absinken, was zwangsläufig eine relativ lange Zeit zur Erholung der Kultur erfordert, bis ein therapeutisch sinnvoller Bereich an Zellkonzentration erneut erreicht ist. Die Neuerung schlägt daher vor, dass bei Indikationen wie bspw. einer polytopen Immunschwäche mit der daraus resultierenden Vielfach-Impfung der Kultur an mehreren Tagen dieses Manko überbrückt wird, indem die Ursprungskultur in mehrere Einzelkulturen aufgeteilt wird, getrennt stimuliert wird und hernach re-integriert wird.

Man kann auf diese Weise ein außerordentlich potentes "Hyperimmun"-Blutpräparat produzieren, das nicht nur sehr gut vertragen wird, sondern aufgrund der Vielfachstimulation auch als pluripotent bezeichnet werden darf.

Eine Nachkultur nach den oben beschriebenen Schritten kann danach über viele Wochen (derzeit Beobachtungszeit bis zur einem viertel Jahr) ohne weitere Stimulation kultiviert werden und erreicht Zellzahlen bis zu 200 000 pro $\mu$l. Aus diesem Pool kann sodann immer wieder "geerntet" werden mit nachfolgender erneuter Anregung des Zellwachstums durch das beschriebene Kulturmedium.

Ebenso wie ein autologes, plurivalentes "Hyperimmun" - Blutpräparat auf diese Weise hergestellt werden kann, kann ein monovalentes oder auch plurivalentes Poolpräparat von mehreren Spendern der gleichen Blutgruppe in diesem Sinne hergestellt werden, das sodann blutgruppenidentischen Empfängern gespritzt werden kann.

Eine besonders gute Verträglichkeit ist zu erwarten bei Präparaten, die nach dem Stand der Technik aus dem zellfreien Überstand oder Zentrifugat derartiger Kulturen gewonnen werden können.

Zusammenfassend wird eine Kultur aus autologem Vollblut vorgeschlagen, das durch ein Kulturmedium genährt und stimuliert wird, das neben den klassischen Bestandteilen an Elektrolyten und Aminosäuren einen hohen Anteil von Phospholipiden vorwiegend pflanzlicher Herkunft, Vitamin E und niedermolekularen Proteoglykanen enthält. Die Eiweißbasis des Kulturmediums besteht aus autologem ozonisiertem Plasma. Die Verwendung eines derartigen Kulturmediums führt zu einem forcierten Zellwachstum. Der dabei beobachtete Überschuss aktivierter Makrophagen wird durch Hydroxyethylstärkelösung von der Reaktionsgefäßwand abgelöst und zu weiteren Nachzuchten verwendet.

Das Endprodukt derartiger Kulturprozesse zeichnet sich durch einen extrem hohen Anteil von Monozyten, basophilen Leukozyten und Thrombozyten aus und bietet sich damit als Heilmittel bei zellulären Immundefekten an.

Die Stimulation dieser Kulturen mit einem Gemisch aus Phospholipiden und Antigenen viraler, bakterieller, mykotischer oder cancerogener Herkunft aber auch mit Allergenen lässt außerdem ihren Einsatz zur spezifischen Abwehr in diesem Sinne oder zur Toleranzentwicklung im Organismus zu. Es wird ein Verfahren zur Auswahl spezifischer Antigene als Stimulanzien für diese Kulturen vorgeschlagen, das auf der Veränderung von haematologischen Parametern des als Kulturgut dienenden Vollblutes beruht.

Das Auswahlverfahren erlaubt die Kombination von Erregern, die sich in ihrem immunmodulatorischen Effekt potenzieren oder auch in ihrer Schadwirkung auf den Organismus gegenseitig hemmen.

Soweit nichts anderes angegeben ist oder sich zwingend aus dem Zusammenhang ergibt beziehen sich sämtliche %-Angaben im Rahmen der vorliegenden Erfindung auf das Gesamtgewicht der Mischung.

**Patentansprüche**

1. Vollblutkultur enthaltend spezifische gegen Tumorzellen, Viren, Bakterien und/oder Allergene aktivierte immunkompetente Zellen, wobei die Vollblutkultur Vollblut und ein Kulturmedium in einem Verhältnis von 3:1 bis 4:1 aufweist, wobei das Kulturmedium

   - einen Sauerstoffüberschuss von mindestens 100 % oder mehr aufweist und
   - ein wasserlösliches Emulgat umfassend ein Gemisch aus Phospholipiden, Vitamin E und niedermolekularen Proteoglykanen mit einem Molekulargewicht von 1200 bis 12000 Dalton enthält und
   wobei tote Tumorzellen oder Fragmente derselben und/oder virale und/oder bakterielle Antigene und/oder Allergene der Vollblutkultur als Antigen beim spezifischen Erkennungsprozess zur Erzeugung der aktivierten Killerzellen (Stimulation) zugesetzt worden sind.

2. Vollblutkultur nach Anspruch 1, **dadurch gekennzeichnet, dass** das Kulturmedium Folsäure enthält.

3. Vollblutkultur nach Anspruch 1 oder 2, **dadurch gekennzeichnet, dass** das Gemisch aus Phospholipiden, Vitamin E und niedermolekularen Proteoglykanen einen Gewichtsanteil von mindestens 10 % des Gewichts des Kulturmediums einnimmt.

4. Vollblutkultur nach einem der Ansprüche 1 bis 3, **dadurch gekennzeichnet, dass** das Vitamin E Alphatocopherol oder Alphatocopherolacetat ist und mit 1 % bis 4 % des Gewichtsanteils des Phospholipidgemisches vorliegt.

5. Vollblutkultur nach einem der Ansprüche 1 bis 4, **dadurch gekennzeichnet, dass** die Vollblutkultur ein Alter von drei bis fünf Tagen hat und

   - einen Anteil von 30 bis 60 Gew% an Monozyten,
   - einen Anteil von > 30 Gew% Basophilen und
   - einen Anteil von > 1 Mio Thrombozyten pro Kubikmillimeter aufweist.

6. Vollblutkultur nach einem der Ansprüche 1 bis 5, **dadurch gekennzeichnet, dass** sie aus mehreren autologen Einzelkulturen zusammengesetzt ist, die mit unterschiedlichen Antigenen stimuliert wurden.

7. Vollblutkultur nach einem der Ansprüche 1 bis 6, **dadurch gekennzeichnet, dass** das Kulturmedium eine Serumbasis aufweist, die aus autologem ozonisiertem Spenderplasma besteht.

8. Vollblutkultur nach einem der Ansprüche 1 bis 6, **dadurch gekennzeichnet, dass** sie ein Poolpräparat aus einzelnen Vollblutkulturen blutgruppenidentischer Spender mit monovalenter oder plurivalenter Stimulierung ist.

9. Verfahren zur Herstellung einer Vollblutkultur enthaltend spezifische gegen Tumorzellen, Viren, Bakterien und/oder Allergene aktivierte Killerzellen, umfassend die Schritte:

   - Kultivieren von Vollblut und einem Kulturmedium aus einem Gemisch aus Phospholipiden, Vitamin E und niedermolekularen Proteoglyanen mit einem Molekulargewicht von 1200 bis 12000 Dalton in einem Verhältnis Vollblut zu Kulturmedium von 3:1 bis 4:1,
   - Zusetzten von toten Tumorzellen oder deren Bruchstücken, und/oder viralen Antigenen und/oder bakteriellen Antigenen und/oder Allergenen als Antigen beim spezifischen Erkennungsprozeß (Stimulation) der sich entwickelnden Abwehrzellen,
- Versetzen des Kulturmediums mit einem Sauerstoffüberschuss von mindestens 100 %.

10. Verfahren nach Anspruch 9, wobei ein wiederholtes Boostern der Vollblutkultur mit einem Gemisch aus Vitamin E, Phospholipiden und/oder Proteoglyanen erfolgt.

11. Verfahren nach Anspruch 10, wobei der Schritt des Boosterns am 2. oder 3. und 5. oder 6. Tag erfolgt.

12. Verfahren nach einem der Ansprüche 10 oder 11, umfassend den Schritt des Ablösens adhaesiver Zellen der Vollblutkultur von einer Wand eines Kultivierungsgefäßes, in dem die Vollblutkultur kultivieren gelassen wird, durch Zugabe einer Haeslösung, insbesondere einer Hydroxyethylstärke.

13. Verfahren nach Anspruch 12, umfassend den Schritt des des mehrfachen Wiederholens der Verfahrensschritte der Ansprüche 9 bis 12 mit wandständigen Zellen.

14. Verfahren nach einem der Ansprüche 9 bis 13, umfassend den Schritt des Stimulierens der Vollblutkultur:

- mit einem von Antigenen aus attenuierten lebenden oder toten Viren, Lyophilisaten aus Bakterien oder Pilzen oder Polysacchariden und sonstigen Fraktionen bakterieller oder mykotischer Herkunft, Fraktionen aus malignen Zellen oder Allergenen tierischer oder pflanzlicher Herkunft oder Kombinationen derselben,
- oder mit einem Gemisch umfassend Virusfraktionen, bakterielle und/oder mykotische Bestandteile und Phospholipide, wobei der Anteil der Phospholipide mindestens 20 % beträgt.

15. Verfahren nach Anspruch 14, umfassend den Schritt des Stimulierens der Vollblutkultur mit einer Kombination verschiedener Antigene viraler, bakterieller und/oder mykotischer Herkunft und/oder mit Allergenen, so dass ein Potenzierungseffekt auf das zelluläre Immunsystem erzielt oder eine Schadwirkung auf einen Organismus durch spezifische Erreger reduziert wird.

16. Verfahren nach einem der Ansprüch 9 bis 15, umfassend den Schritt des Stimulierens der Vollblutkultur mit einem Gemisch von Antigenen zu unterschiedlichen Zeitpunkten nacheinander, vorzugsweise zu einem Zeitpunkt, wenn eine maximale Proliferation der Zellpopulationen erfolgt.

17. Verfahren nach einem der Ansprüche 9 bis 16, **dadurch gekennzeichnet, dass** ein autologes oder homologes zellfreies Serum aus einem Überstand oder Zentrifugat der Vollblutkultur gewonnen wird.

18. Heilmittel zur Immunstimulierung oder Behandlung von Immundefekten oder malignen Erkrankungen, **dadurch gekennzeichnet, dass** es eine Vollblutkultur nach einem der Ansprüche 1-8 oder ein zellfreies Serum aus einem Überstand oder Zentrifugat der Vollblutkultur nach einem der Ansprüche 1-8 ist.

## Claims

1. Whole-blood culture containing specific immunocompetent cells that are activated against tumor cells, viruses, bacteria and/or allergens, wherein the whole-blood culture contains whole-blood and a culture medium in a ratio of 3:1 to 4:1, wherein the culture medium contains

- an oxygen excess of at least 100% or more, and
- a water-soluble emulsification product comprising a mixture of phospholipids, vitamin E, and low-molecular weight proteoglycans with a molecular weight of 1,200 to 12,000 Dalton, and
wherein dead tumor cells or fragments thereof and/or viral and/or bacterial antigens and/or allergens have been added to the whole-blood culture as antigen during the specific recognition process for production of the activated killer cells (stimulation).

2. Whole-blood culture according to claim 1, **characterized in that** the culture medium contains folic acid.

3. Whole-blood culture according to claim 1 or 2, **characterized in that** the mixture of phospholipids, vitamin E, and low-molecular weight proteoglycans accounts for a weight fraction of at least 10 % of the weight of the culture medium.

4. Whole-blood culture according to any one of the claims 1 to 3, **characterized in that** the vitamin E is alpha-tocopherol or alpha-tocopherol acetate and is present in a weight fraction of 1 % to 4 % of the phospholipid mixture.

5. Whole-blood culture according to any one of the claims 1 to 4, **characterized in that** the whole-blood culture has been cultured for three to five days and has

- a monocyte fraction of 30 to 60 wt-%;
- a basophilic granulocyte fraction of > 30 wt-%; and
- a platelet fraction of > 1 million per cubic-millimeter.

6. Whole-blood culture according to any one of the claims 1 to 5, **characterized in that** it is composed of multiple autologous individual cultures that have been stimulated with different antigens.

7. Whole-blood culture according to any one of the claims 1 to 6, **characterized in that**, the whole-blood culture, the culture medium has a serum base that consists of autologous ozonized donor plasma.

8. Whole-blood culture according to any one of the claims 1 to 6, **characterized in that** it is a pool preparation of individual whole-blood cultures from donors of identical blood type with monovalent or plurivalent stimulation.

9. Method for producing a whole-blood culture containing specific killer cells that are activated against tumor cells, viruses, bacteria and/or allergens, comprising the steps:

- culturing of whole-blood and a culture medium made up of a mixture of phospholipids, vitamin E, and low-molecular weight proteoglycans with a molecular weight of 1,200 to 12,000 Dalton at a whole-blood-to-culture medium ratio of 3:1 to 4:1;
- addition of dead tumor cells or fragments thereof and/or viral antigens and/or bacterial antigens and/or allergens as antigen during the specific recognition process (stimulation) of the developing defense cells,
- supplying an oxygen excess of at least 100% to the culture medium.

10. Method according to claim 9, wherein repeated boostering of the whole-blood culture with a mixture of vitamin E, phospholipids and/or proteoglycans is carried out.

11. Method according to claim 10, wherein the step of boostering is carried out on day 2 or 3 and on day 5 or 6.

12. Method according to any one of the claims 10 to 11, comprising the step of detaching adhesive cells of the whole-blood culture from a wall of a culture vessel, in which the whole-blood culture is cultured, by adding a Haes solution, in particular a hydroxyethyl-starch.

13. Method according to claim 12, comprising the step of multiply repeating the procedural steps of claims 9 to 12 several times with cells residing on the wall.

14. Method according to any one of the claims 9 to 13, comprising the step of stimulating the whole-blood culture:

- with one of antigens of attenuated life or dead viruses, lyophilization products of bacteria or fungi or polysaccharides and other fractions of bacterial or mycotic origin, fractions from malignant cells or allergens of animal or plant origin or combinations thereof,
- or with a mixture comprising virus fractions, bacterial and/or mycotic components and phospholipids, wherein the fraction of phospholipids is at least 20%.

15. Method according to claim 14, comprising the step of stimulating the whole-blood culture with a combination of various antigens of viral, bacterial and/or mycotic origin and/or with allergens such that a potentiating effect on the cellular immune system is achieved or a damaging effect of specific pathogens on an organism is reduced.

16. Method according to any one of the claims 9 to 15, comprising the step of stimulating the whole-blood culture with a mixture of antigens at different sequential points in time, preferably at a point in time at which maximal proliferation of the cell population occurs.

17. Method according to any one of the claims 9 to 16, **characterized in that** an autologous or homologous cell-free serum is obtained from a supernatant or centrifugation product of the whole-blood culture.

**18.** Medicinal agent for immunostimulation or treatment of immune defect or malignant diseases, **characterized in that** it is a whole-blood culture according to any one of the claims 1-8 or a cell-free serum from a supernatant or centrifugation product of the whole-blood culture according to any one of the claims 1-8.

**Revendications**

**1.** Produit de culture de sang total, contenant des cellules immunocompétentes spécifiques activées contre des cellules tumorales, des virus, des bactéries et/ou des allergènes, ledit produit de culture de sang total comportant du sang total et un milieu de culture dans un rapport compris entre 3 : 1 et 4 : 1, ledit milieu de culture

- présentant un excédant d'oxygène supérieur ou égal à 100 %, et
- contenant un produit émulsifié hydrosoluble lequel comprend un mélange de phospholipides, de vitamine E et de protéoglycanes à faible masse moléculaire dont la masse moléculaire est comprise entre 1 200 et 12 000 dalton, et
ledit produit de culture sang total comportant des cellules tumorales mortes ou des fragments de celles-ci et/ou des antigènes viraux et/ou bactériens et/ou des allergènes lesquels y ont été ajoutés à titre d'antigènes destinés à produire, lors du processus de reconnaissance spécifique, des cellules tueuses activées (stimulation).

**2.** Produit de culture de sang total selon la revendication 1, **caractérisé en ce que** ledit milieu de culture contient de l'acide folique.

**3.** Produit de culture de sang total selon les revendications 1 ou 2, **caractérisé en ce que** ledit mélange de phospholipides, de vitamine E et de protéoglycanes à faible masse moléculaire représente une proportion pondérale d'au moins 10 % par rapport au poids du milieu de culture.

**4.** Produit de culture de sang total selon l'une des revendications 1 à 3, **caractérisé en ce que** ladite vitamine E est de l'alpha-tocophérol ou de l'acétate d'alpha-tocophérol et qu'elle représente une proportion pondérale comprise entre 1 % et 4 % par rapport au mélange de phospholipides.

**5.** Produit de culture de sang total selon l'une des revendications 1 à 4, **caractérisé en ce que** l'âge dudit produit de culture de sang total est compris entre trois et cinq jours, et qu'il comporte

- une proportion de monocytes comprise entre 30 et 60 % en poids,
- une proportion de basophiles > 30 % en poids, et
- une proportion de thrombocytes correspondant à > 1 million par millimètre cube.

**6.** Produit de culture de sang total selon l'une des revendications 1 à 5, **caractérisé en ce qu'**il est composé de plusieurs cultures autologues individuelles lesquelles ont été stimulées au moyen de différents antigènes.

**7.** Produit de culture de sang total selon l'une des revendications 1 à 6, **caractérisé en ce que** ledit milieu de culture comporte un sérum de base qui est constitué de plasma autologue issu d'un don et traité à l'ozone.

**8.** Produit de culture de sang total selon l'une des revendications 1 à 6, **caractérisé en ce qu'**il est préparé en réunissant des cultures de sang total individuelles issues de donneurs d'un même groupe sanguin et ayant subi une stimulation monovalente ou plurivalente.

**9.** Procédé qui est destiné à obtenir un produit de culture de sang total contenant des cellules tueuses spécifiques activées contre des cellules tumorales, des virus, des bactéries et/ou des allergènes et qui comprend les étapes suivantes :

- mise en culture de sang total et d'un milieu de culture réalisé à partir d'un mélange de phospholipides, de vitamine E et de protéoglycanes à faible masse moléculaire dont la masse moléculaire est comprise entre 1 200 et 12 000 dalton, le rapport entre le sang total et ledit milieu de culture étant compris entre 3 : 1 et 4 : 1,
- ajout de cellules tumorales mortes ou de fragments de celles-ci, et/ou d'antigènes viraux et/ou d'antigènes bactériens et/ou d'allergènes, destinés à jouer le rôle d'antigène lors du processus de reconnaissance spécifique (stimulation) des cellules de défense en cours de développement,
- ajout d'oxygène audit milieu de culture, de manière à en obtenir un excédent d'au moins 100 %.

10. Procédé selon la revendication 9, le produit de culture de sang total étant, à plusieurs reprises, réapprovisionné en un mélange de vitamine E, de phospholipides et/ou de protéoglycanes.

11. Procédé selon la revendication 10, l'étape de réapprovisionnement étant mise en oeuvre le 2ème ou le 3ème ainsi que le 5ème ou le 6ème jour.

12. Procédé selon l'une des revendications 10 et 11, comprenant une étape consistant à détacher, au sein de la culture de sang total, des cellules adhérant à une paroi du récipient de culture dans lequel on met en oeuvre la culture de sang total, par ajout d'une solution d'HEA, notamment d'un hydroxyéthylamidon.

13. Procédé selon la revendication 12, comprenant l'étape consistant à répéter plusieurs fois les étapes de procédé selon les revendications 9 à 12, en les appliquant aux cellules adhérant aux parois.

14. Procédé selon l'une des revendications 9 à 13, comprenant l'étape consistant à stimuler la culture de sang total en mettant en oeuvre l'un des agents suivants :

   - antigènes issus de virus vivants atténués ou morts, lyophilisats de bactéries ou de champignons ou de polysaccharides et d'autres fractions d'origine bactérienne ou fongique, fractions issus de cellules malignes, ou allergènes d'origine animale ou végétale, ou une combinaison des éléments précités, ou bien
   - un mélange comprenant des fractions de virus, des constituants bactériens et/ou fongiques, et des phospholipides, la proportion des phospholipides étant supérieure ou égale à 20 %.

15. Procédé selon la revendication 14, comprenant l'étape consistant à stimuler le produit de culture de sang total en mettant en oeuvre une combinaison de différents antigènes d'origine virale, bactérienne et/ou fongique avec des allergènes, pour ainsi potentialiser l'effet sur le système immunitaire cellulaire ou pour réduire des effets nocifs qu'un organisme peut subir en raison d'agents spécifiques.

16. Procédé selon l'une des revendications 9 à 15, comprenant l'étape consistant à stimuler la culture de sang total en mettant en oeuvre un mélange d'antigènes, ces derniers étant mis en oeuvre successivement à différents moments, de préférence à un moment correspondant à la prolifération maximale de la population de cellules.

17. Procédé selon l'une des revendications 9 à 16, **caractérisé en ce que** l'on récupère un sérum autologue ou homologue exempt de cellules, à partir d'un surnageant, obtenu éventuellement après centrifugation, du produit de culture de sang total.

18. Médicament destiné à l'immunostimulation ou au traitement de déficiences immunitaires ou d'affections malignes, **caractérisé en ce qu'**il s'agit d'un produit de culture de sang total selon l'une des revendications 1 à 8 ou d'un sérum exempt de cellules qui est issu d'un surnageant, obtenu éventuellement après centrifugation, du produit de culture de sang total selon l'une des revendications 1 à 8.

Figur 1

**Entwicklung des Differentialblutbildes von Kulturen ohne Phospholipide und
Alphatocopherol (Patienten mit malignen Erkrankungen)**

Lympho = Lymphozyten    MPXI = Myeloperoxidase positive Zellen        Neut = Neutrozyten
Eos = Eosinophile Granulozyten        Baso = Basophile Granulozyten        Mono = Monozyten
Luc = Large unstained cells = große ungefärbte Zellen

Figur 2

**Entwicklung des Differentialblutbildes von Kulturen nach dem beschriebenen Verfahren**
**(Patienten mit malignen Erkrankungen)**

Figur 3

Thrombozytenvergleich

Thrombozyten: x 1000 ml²

Nativ | 1. Tag | 2. Tag | 3. Tag | 4. Tag | 5. Tag

2500 2000 1500 1000 500 0

◆ Kulturmedium ohne Vit. E + Phospholipiden

■ Kulturmedium mit Vit. E + Phospholipiden

n = 19

Figur 4

**Entwicklung der Leukozytenmengen bei dem beschriebenen Verfahren (Nachkultur)**
**Zugabe des Phospholipid-Vitamin E - Gemischs am 3. und 5. Tag**

Leukozyten x 1000 / Mikroliter

n = 20

Figur 5

**Entwicklung des Differentialblutbildes einer
Nachkultur aus aktivierten Makrophagen nach dem beschriebenen Verfahren**

EP 2 120 975 B1

Figur 6 a

**Kumulativdarstellung immunmodulatorischer Effekte auf das Blut von Neurodermitispatienten
durch verschiedene Toxine und Antigene
Δ% Granularität: Menge an anfärbbarem Biomaterial von Granulozyten und Lymphozyten
(Neut x)**

n = 34

Figur 6 b

**Kumulativdarstellung immunmodulatorischer Effekte auf das Blut von Neurodermitispatienten
durch verschiedene Toxine u. Antigene
Δ% RNA/DNA-Gehalt von Granulozyten und Lymphozyten
(Neut y)**

n = 28

Figur 6c

Kumulativdarstellung immunmodulatorischer Effekte auf das Blut von Neurodermitispatienten
durch verschiedene Toxine u. Antigene
Toxizitätseffekte: Δ% Zellvolumen
(IMI DC)

EP 2 120 975 B1

n = 34

Figur 6 d

**Kulturtransformationstest**
**Kumulativstatistik - Neurodermitis**
**Kulturtransformationstest - SF1: Δ% Zellmorphologie der Granulozyten**

EP 2 120 975 B1

Figur 6 e

**Kulturtransformationstest**
**Kumulativstatistik - Neurodermitis**
**Kulturtransformationstest - SF3: Δ% von Zellmorphologie und Zellzahl**

n = 31

Figur 6 f

**Kumulativdarstellung immunmodulatorischer Effekte auf das Blut von Neurodermitispatienten
durch verschiedene Toxine u. Antigene
Kulturtransformationstest - SF4:  Δ% Granulozyten x  Δ% Lymphozyten**

n = 29

EP 2 120 975 B1

**Kulturtransformationstest
Fall von Multipler Sklerose
IMI DC**

Figur 7 a

Figur 7 b

**Kulturtransformationstest - SF3**
**Fall von Multipler Sklerose**

Figur 7 c

**Kulturtransformationstest - SF4**
**Fall von Multiple Sklerose**

EP 2 120 975 B1

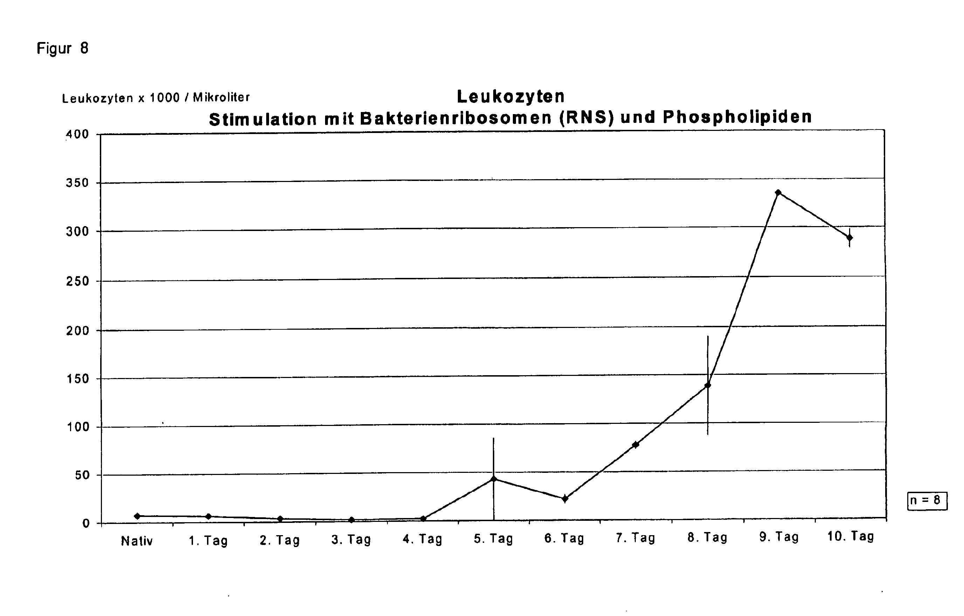

Figur 8

Figur 8: Leukozyten x 1000 / Mikroliter — Leukozyten — Stimulation mit Bakterienribosomen (RNS) und Phospholipiden

n = 8

EP 2 120 975 B1

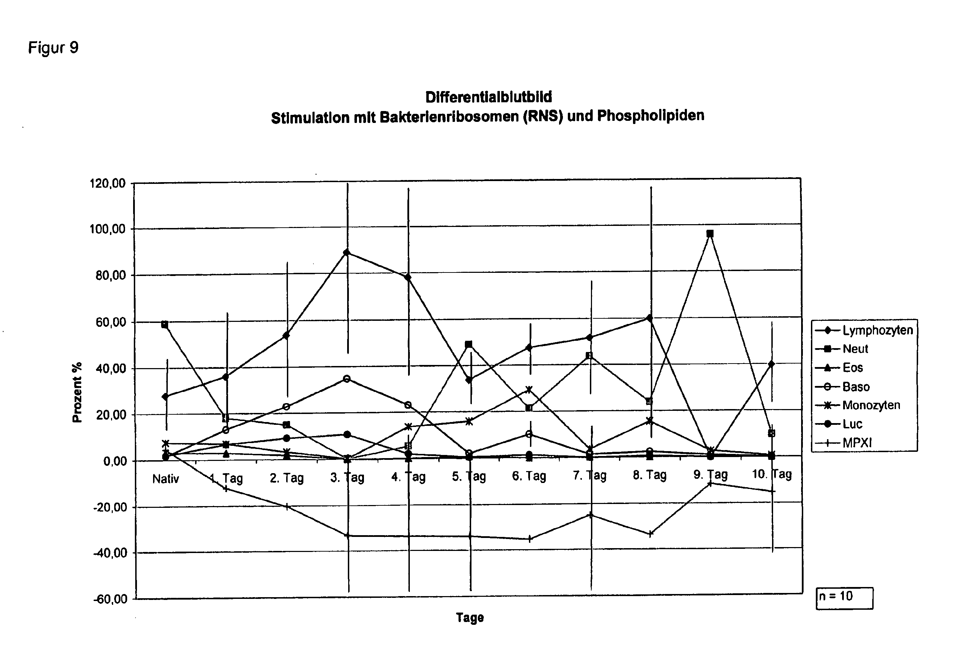

Figur 9

**Differentialblutbild**
**Stimulation mit Bakterienribosomen (RNS) und Phospholipiden**

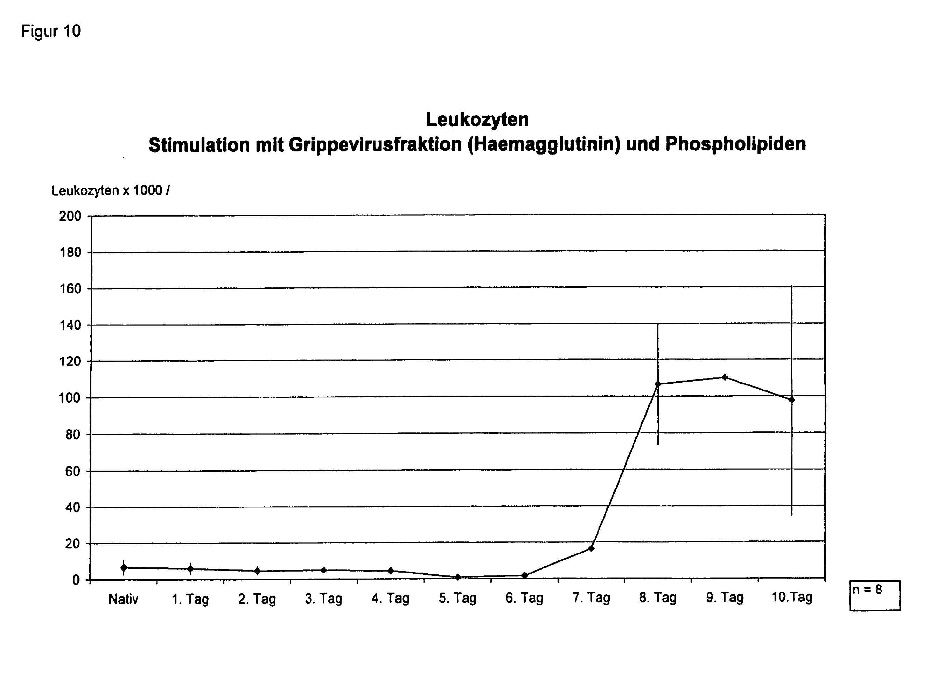

Figur 10

**Leukozyten**
**Stimulation mit Grippevirusfraktion (Haemagglutinin) und Phospholipiden**

Leukozyten x 1000 /

EP 2 120 975 B1

Figur 11

Differentialblutbild
Stimulation mit Grippevirusfraktion (Haemagglutinin) und Phospholipiden

EP 2 120 975 B1

Figur 12

**Leukozyten**
**Stimulation mit Grippevirusfraktion (Haemagglutinin)**
**Bakterienribosomen (RNS) und Phospholipiden**

n = 9

Leukozyten x 1000 / Mikroliter

400 — 350 — 300 — 250 — 200 — 150 — 100 — 50 — 0

Nativ  1. Tag  2. Tag  3. Tag  4. Tag  5. Tag  6. Tag  7. Tag  8. Tag  9. Tag  10. Tag

30

Figur 13

**Differentialblutbild**
**Stimulation mit Grippevirusfraktion (Haemagglutinin)**
**Bakterienribosomen (RNS) und Phospholipiden**

EP 2 120 975 B1

n = 9

**Tage**

Figur 14

Leukozyten
Nachkultur

n = 19

Differentialblutbild
Nachkultur

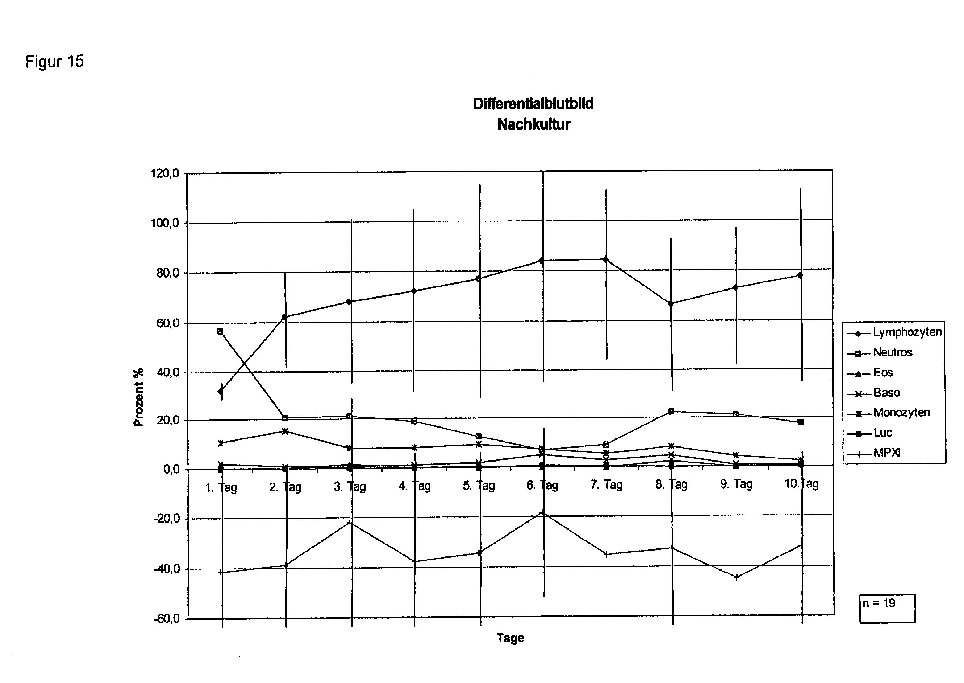

Figur 15

n = 19